# EUROPÄISCHE PATENTSCHRIFT

(11) **EP 0 729 580 B1**
(45) Veröffentlichungstag und Bekanntmachung des Hinweises auf die Patenterteilung: **02.02.2000**
(21) Anmeldenummer: 95902774.9
(22) Anmeldetag: 18.11.1994
(51) Int. Cl.: G01N 33/574, G01N 33/543, C07K 14/47, C12N 15/12

(54) **NACHWEISVERFAHREN FÜR HDM-2-SPEZIFISCHE ANTIKÖRPER**
METHOD OF IDENTIFYING HDM-2-SPECIFIC ANTIBODIES
PROCEDE D'IDENTIFICATION D'ANTICORPS SPECIFIQUES CONTRE L'HDM-2

(30) Priorität: 19.11.1993 DE 4339533
(43) Veröffentlichungstag der Anmeldung: 04.09.1996
(73) Patentinhaber: Deutsches Krebsforschungszentrum Stiftung des öffentlichen Rechts, 69120 Heidelberg (DE)
(72) Erfinder: ZENTGRAF, Hanswalter, D-69155 Heidelberg (DE); KLEIN, Ralf, D-68259 Mannheim (DE); FREY, Manfred, D-68259 Mannheim (DE); MARTENS, Regina, D-68799 Reilingen (DE)
(74) Vertreter: Schüssler, Andrea, Dr.
(86) Internationale Anmeldenummer: EP9403823
(87) Internationale Veröffentlichungsnummer: WO9514233

(56) Entgegenhaltungen:
- WO-A-93/20238
- ONCOGENE (1993), 8(9), 2353-60 CODEN: ONCNES;ISSN: 0950-9232, 1993 OLSON, DAVID C. ET AL 'Identification and characterization of multiple mdm - proteins and mdm - 2 -p53 protein complexes'
- NATURE., Bd.358, 2. Juli 1992, LONDON GB Seiten 80 - 83 J.D. OLINER, K.W. KINZLER, P.S. MELTZER, D.L. GEORGE & B. VOGELSTEIN 'Amplification of a gene encoding a p53-associated protein in human sarcomas' in der Anmeldung erwähnt
- 168TH MEETING OF THE PATHOLOGICAL SOCIETY OF GREAT BRITAIN AND IRELAND, LONDON, ENGLAND, UK, JANUARY 5-7, 1994. JOURNAL OF PATHOLOGY 172 (SUPPL.). 1994. 135A. ISSN: 0022-3417 ANDERSON J J ET AL 'Production and characterisation of monoclonal antibodies for use in immunohistochemical studies of the homologue of the rine double minute 2 gene product ( MDM2'
- Clinical Biochemistry 25, 1992, 445 - 449
- Methods in Enzymology 70, 1980, Hg. H. van Vunakis & J. Langone, pp. 419, 420, 434 - 438

### SEQUENZPROTOKOLL

### (1) ALLGEMEINE ANGABEN:

(i) ANMELDER:
   (A) NAME: Deutsches Krebsforschungszentrum Stiftung des öffentlichen Rechts
   (B) STRASSE: Im Neuenheimer Feld 280
   (C) ORT: Heidelberg
   (E) LAND:Deutschland
   (F) POSTLEITZAHL: 69009
   (G) TELEFON: 06221/42-0
(ii) BEZEICHNUNG DER ERFINDUNG: Nachweisverfahren für hdm-2 spezifische Antikörper
(iii) ANZAHL DER SEQUENZEN: 10
(iv) COMPUTERLESBARE FASSUNG:
   (A) DATENTRÄGER: Diskette
   (B) COMPUTER: IBM PC kompatibel
   (C) BETRIEBSSYSTEM: MS-DOS
   (D) SOFTWARE: Word Perfect, konvertiert in ASCii Codes
(v) DATEN DER JETZIGEN ANMELDUNG:
   ANMELDENUMMER: PCT/EP/94/03823
(vi) DATEN DER VORANMELDUNG:
   (A) ANMELDENUMMER: DE P 43 39 533.3
   (B) ANMELDETAG: 19. November 1993

### (2) ANGABEN ZU SEQ ID NO: 1:

(i) SEQUENZKENNZEICHEN:
   (A) LÄNGE: 284 Aminosäuren
   (B) ART: Aminosäure
   (D) TOPOLOGIE: linear
(ii) ART DES MOLEKÜLS: Protein
(v) ART DES FRAGMENTS: N-Terminus
(xi) SEQUENZBESCHREIBUNG: SEQ ID NO: 1:
   Met Cys Asn Thr Asn Met Ser Val Pro Thr Asp Gly Ala Val Thr Thr
     1               5                  10                  15
   Ser Gln Ile Pro Ala Ser Glu Gln Glu Thr Leu Val Arg Pro Lys Pro
               20                  25                  30
   Leu Leu Leu Lys Leu Leu Lys Ser Val Gly Ala Gln Lys Asp Thr Tyr
           35                  40                  45
   Thr Met Lys Glu Val Leu Phe Tyr Leu Gly Gln Tyr Ile Met Thr Lys
       50                  55                  60
   Arg Leu Tyr Asp Glu Lys Gln Gln His Ile Val Tyr Cys Ser Asn Asp
    65                  70                  75                  80
   Leu Leu Gly Asp Leu Phe Gly Val Pro Ser Phe Ser Val Lys Glu His
                   85                  90                  95
   Arg Lys Ile Tyr Thr Met Ile Tyr Arg Asn Leu Val Val Val Asn Gln
               100                 105                 110
   Gln Glu Ser Ser Asp Ser Gly Thr Ser Val Ser Glu Asn Arg Cys His
           115                 120                 125
   Leu Glu Gly Gly Ser Asp Gln Lys Asp Leu Val Gln Glu Leu Gln Glu
       130                 135                 140
   Glu Lys Pro Ser Ser Ser His Leu Val Ser Arg Pro Ser Thr Ser Ser
   145                 150                 155                 160
   Arg Arg Arg Ala Ile Ser Glu Thr Glu Glu Asn Ser Asp Glu Leu Ser
                   165                 170                 175
   Gly Glu Arg Gln Arg Lys Arg His Lys Ser Asp Ser Ile Ser Leu Ser
               180                 185                 190
   Phe Asp Glu Ser Leu Ala Leu Cys Val Ile Arg Glu Ile Cys Cys Glu
           195                 200                 205
   Arg Ser Ser Ser Ser Glu Ser Thr Gly Thr Pro Ser Asn Pro Asp Leu
       210                 215                 220
   Asp Ala Gly Val Ser Glu His Ser Gly Asp Trp Leu Asp Gln Asp Ser
   225                 230                 235                 240
   Val Ser Asp Gln Phe Ser Val Glu Phe Glu Val Glu Ser Leu Asp Ser
                   245                 250                 255
   Glu Asp Tyr Ser Leu Ser Glu Glu Gly Gln Glu Leu Ser Asp Glu Asp
               260                 265                 270
   Asp Glu Val Tyr Gln Val Thr Val Tyr Gln Ala Gly
           275                 280

### (2) ANGABEN ZU SEQ ID NO: 2:

(i) SEQUENZKENNZEICHEN:
   (A) LÄNGE: 227 Aminosäuren
   (B) ART: Aminosäure
   (D) TOPOLOGIE: linear
(ii) ART DES MOLEKÜLS: Protein
(v) ART DES FRAGMENTS: inneres Fragment
(xi) SEQUENZBESCHREIBUNG: SEQ ID NO: 2:

### (2) ANGABEN ZU SEQ ID NO: 3:

(i) SEQUENZKENNZEICHEN:
   (A) LÄNGE: 434 Aminosäuren
   (B) ART: Aminosäure
   (D) TOPOLOGIE: linear
(ii) ART DES MOLEKÜLS: Protein
(v) ART DES FRAGMENTS: C-Terminus
(xi) SEQUENZBESCHREIBUNG: SEQ ID NO: 3:
   Gly Gln Tyr Ile Met Thr Lys Arg Leu Tyr Asp Glu Lys Gln Gln His
    1               5                  10                  15
   Ile Val Tyr Cys Ser Asn Asp Leu Leu Gly Asp Leu Phe Gly Val Pro
               20                  25                  30
   Ser Phe Ser Val Lys Glu His Arg Lys Ile Tyr Thr Met Ile Tyr Arg
           35                  40                  45
   Asn Leu Val Val Val Asn Gln Gln Glu Ser Ser Asp Ser Gly Thr Ser
               50                  55                  60
   Val Ser Glu Asn Arg Cys His Leu Glu Gly Gly Ser Asp Gln Lys Asp
   65                  70                  75                  80
   Leu Val Gln Glu Leu Gln Glu Glu Lys Pro Ser Ser Ser His Leu Val
                   85                  90                  95
   Ser Arg Pro Ser Thr Ser Ser Arg Arg Arg Ala Ile Ser Glu Thr Glu
               100                 105                 110
   Glu Asn Ser Asp Glu Leu Ser Gly Glu Arg Gln Arg Lys Arg His Lys
           115                 120                 125
   Ser Asp Ser Ile Ser Leu Ser Phe Asp Glu Ser Leu Ala Leu Cys Val
       130                 135                 140
   Ile Arg Glu Ile Cys Cys Glu Arg Ser Ser Ser Ser Glu Ser Thr Gly
   145                 150                 155                 160
   Thr Pro Ser Asn Pro Asp Leu Asp Ala Gly Val Ser Glu His Ser Gly
                   165                 170                 175
   Asp Trp Leu Asp Gln Asp Ser Val Ser Asp Gln Phe Ser Val Glu Phe
               180                 185                 190
   Glu Val Glu Ser Leu Asp Ser Glu Asp Tyr Ser Leu Ser Glu Glu Gly
           195                 200                 205
   Gln Glu Leu Ser Asp Glu Asp Asp Glu Val Tyr Gln Val Thr Val Tyr
       210                 215                 220
   Gln Ala Gly Glu Ser Asp Thr Asp Ser Phe Glu Glu Asp Pro Glu Ile
   225                 230                 235                 240
   Ser Leu Ala Asp Tyr Trp Lys Cys Thr Ser Cys Asn Glu Met Asn Pro
                   245                 250                 255
   Pro Leu Pro Ser His Cys Asn Arg Cys Trp Ala Leu Arg Glu Asn Trp
               260                 265                 270
   Leu Pro Glu Asp Lys Gly Lys Asp Lys Gly Glu Ile Ser Glu Lys Ala
           275                 280                 285
   Lys Leu Glu Asn Ser Thr Gln Ala Glu Glu Gly Phe Asp Val Pro Asp
       290                 295                 300
   Cys Lys Lys Thr Ile Val Asn Asp Ser Arg Glu Ser Cys Val Glu Glu
   305                 310                 315                 320
   Asn Asp Asp Lys Ile Thr Gln Ala Ser Gln Ser Gln Glu Ser Glu Asp
                   325                 330                 335
   Tyr Ser Gln Pro Ser Thr Ser Ser Ser Ile Ile Tyr Ser Ser Gln Glu
               340                 345                 350
   Asp Val Lys Glu Phe Glu Arg Glu Glu Thr Gln Asp Lys Glu Glu Ser
           355                 360                 365
   Val Glu Ser Ser Leu Pro Leu Asn Ala Ile Glu Pro Cys Val Ile Cys
       370                 375                 380
   Gln Gly Arg Pro Lys Asn Gly Cys Ile Val His Gly Lys Thr Gly His
   385                 390                 395                 400
   Leu Met Ala Cys Phe Thr Cys Ala Lys Lys Leu Lys Lys Arg Asn Lys
                   405                 410                 415
   Pro Cys Pro Val Cys Arg Gln Pro Ile Gln Met Ile Val Leu Thr Tyr
               420                 425                 430
   Phe Pro

### (2) ANGABEN ZU SEQ ID NO: 4:

(i) SEQUENZKENNZEICHEN:
   (A) LÄNGE: 852 Basenpaare
   (B) ART: Nukleinsäure
   (C) STRANGFORM: Einzelstrang
   (D) TOPOLOGIE: linear
(ii) ART DES MOLEKÜLS: cDNA
(xi) SEQUENZBESCHREIBUNG: SEQ ID NO: 4:
   ATGTGCAATA CCAACATGTC TGTACCTACT GATGGTGCTG TAACCACCTC ACAGATTCCA    60
   GCTTCGGAAC AAGAGACCCT GGTTAGACCA AAGCCATTGC TTTTGAAGTT ATTAAAGTCT   120
   GTTGGTGCAC AAAAAGACAC TTATACTATG AAAGAGGTTC TTTTTTATCT TGGCCAGTAT   180
   ATTATGACTA AACGATTATA TGATGAGAAG CAACAACATA TTGTATATTG TTCAAATGAT   240
   CTTCTAGGAG ATTTGTTTGG CGTGCCAAGC TTCTCTGTGA AAGAGCACAG GAAAATATAT   300
   ACCATGATCT ACAGGAACTT GGTAGTAGTC AATCAGCAGG AATCATCGGA CTCAGGTACA   360
   TCTGTGAGTG AGAACAGGTG TCACCTTGAA GGTGGGAGTG ATCAAAAGGA CCTTGTACAA   420
   GAGCTTCAGG AAGAGAAACC TTCATCTTCA CATTTGGTTT CTAGACCATC TACCTCATCT   480
   AGAAGGAGAG CAATTAGTGA GACAGAAGAA AATTCAGATG AATTATCTGG TGAACGACAA   540
   AGAAAACGCC ACAAATCTGA TAGTATTTCC CTTTCCTTTG ATGAAAGCCT GGCTCTGTGT   600
   GTAATAAGGG AGATATGTTG TGAAAGAAGC AGTAGCAGTG AATCTACAGG GACGCCATCG   660
   AATCCGGATC TTGATGCTGG TGTAAGTGAA CATTCAGGTG ATTGGTTGGA TCAGGATTCA   720
   GTTTCAGATC AGTTTAGTGT AGAATTTGAA GTTGAATCTC TCGACTCAGA AGATTATAGC   780
   CTTAGTGAAG AAGGACAAGA ACTCTCAGAT GAAGATGATG AGGTATATCA AGTTACTGTG   840
   TATCAGGCAG GG                                                       852

### (2) ANGABEN ZU SEQ ID NO: 5:

(i) SEQUENZKENNZEICHEN:
   (A) LÄNGE: 681 Basenpaare
   (B) ART: Nukleinsäure
   (C) STRANGFORM: Einzelstrang
   (D) TOPOLOGIE: linear
(ii) ART DES MOLEKÜLS: cDNA
(xi) SEQUENZBESCHREIBUNG: SEQ ID NO: 5:
   GGCCAGTATA TTATGACTAA ACGATTATAT GATGAGAAGC AACAACATAT TGTATATTGT    60
   TCAAATGATC TTCTAGGAGA TTTGTTTGGC GTGCCAAGCT TCTCTGTGAA AGAGCACAGG   120
   AAAATATATA CCATGATCTA CAGGAACTTG GTAGTAGTCA ATCAGCAGGA ATCATCGGAC   180
   TCAGGTACAT CTGTGAGTGA GAACAGGTGT CACCTTGAAG GTGGGAGTGA TCAAAAGGAC   240
   CTTGTACAAG AGCTTCAGGA AGAGAAACCT TCATCTTCAC ATTTGGTTTC TAGACCATCT   300
   ACCTCATCTA GAAGGAGAGC AATTAGTGAG ACAGAAGAAA ATTCAGATGA ATTATCTGGT   360
   GAACGACAAA GAAAACGCCA CAAATCTGAT AGTATTTCCC TTTCCTTTGA TGAAAGCCTG    420
   GCTCTGTGTG TAATAAGGGA GATATGTTGT GAAAGAAGCA GTAGCAGTGA ATCTACAGGG    480
   ACGCCATCGA ATCCGGATCT TGATGCTGGT GTAAGTGAAC ATTCAGGTGA TTGGTTGGAT    540
   CAGGATTCAG TTTCAGATCA GTTTAGTGTA GAATTTGAAG TTGAATCTCT CGACTCAGAA    600
   GATTATAGCC TTAGTGAAGA AGGACAAGAA CTCTCAGATG AAGATGATGA GGTATATCAA    660
   GTTACTGTGT ATCAGGCAGG G                                              681

### (2) ANGABEN ZU SEQ ID NO: 6:

(i) SEQUENZKENNZEICHEN:
   (A) LÄNGE: 1302 Basenpaare
   (B) ART: Nukeleinsäure
   (C) STRABGFORM: Einzelstrang
   (D) TOPOLOGIE: linear
(ii) ART DES MOLEKÜLS: cDNA
(xi) SEQUENZBESCHREIBUNG: SEQ ID NO: 6:
   GGCCAGTATA TTATGACTAA ACGATTATAT GATGAGAAGC AACAACATAT TGTATATTGT     60
   TCAAATGATC TTCTAGGAGA TTTGTTTGGC GTGCCAAGCT TCTCTGTGAA AGAGCACAGG    120
   AAAATATATA CCATGATCTA CAGGAACTTG GTAGTAGTCA ATCAGCAGGA ATCATCGGAC    180
   TCAGGTACAT CTGTGAGTGA GAACAGGTGT CACCTTGAAG GTGGGAGTGA TCAAAAGGAC    240
   CTTGTACAAG AGCTTCAGGA AGAGAAACCT TCATCTTCAC ATTTGGTTTC TAGACCATCT    300
   ACCTCATCTA GAAGGAGAGC AATTAGTGAG ACAGAAGAAA ATTCAGATGA ATTATCTGGT    360
   GAACGACAAA GAAAACGCCA CAAATCTGAT AGTATTTCCC TTTCCTTTGA TGAAAGCCTG    420
   GCTCTGTGTG TAATAAGGGA GATATGTTGT GAAAGAAGCA GTAGCAGTGA ATCTACAGGG    480
   ACGCCATCGA ATCCGGATCT TGATGCTGGT GTAAGTGAAC ATTCAGGTGA TTGGTTGGAT    540
   CAGGATTCAG TTTCAGATCA GTTTAGTGTA GAATTTGAAG TTGAATCTCT CGACTCAGAA    600
   GATTATAGCC TTAGTGAAGA AGGACAAGAA CTCTCAGATG AAGATGATGA GGTATATCAA    660
   GTTACTGTGT ATCAGGCAGG GGAGAGTGAT ACAGATTCAT TTGAAGAAGA TCCTGAAATT    720
   TCCTTAGCTG ACTATTGGAA ATGCACTTCA TGCAATGAAA TGAATCCCCC CCTTCCATCA    780
   CATTGCAACA GATGTTGGGC CCTTCGTGAG AATTGGCTTC CTGAAGATAA AGGGAAAGAT    840
   AAAGGGGAAA TCTCTGAGAA AGCCAAACTG GAAAACTCAA CACAAGCTGA AGAGGGCTTT    900
   GATGTTCCTG ATTGTAAAAA AACTATAGTG AATGATTCCA GAGAGTCATG TGTTGAGGAA    960
   AATGATGATA AAATTACACA AGCTTCACAA TCACAAGAAA GTGAAGACTA TTCTCAGCCA   1020
   TCAACTTCTA GTAGCATTAT TTATAGCAGC CAAGAAGATG TGAAAGAGTT TGAAAGGGAA   1080
   GAAACCCAAG ACAAAGAAGA GAGTGTGGAA TCTAGTTTGC CCCTTAATGC CATTGAACCT   1140
   TGTGTGATTT GTCAAGGTCG ACCTAAAAAT GGTTGCATTG TCCATGGCAA AACAGGACAT   1200
   CTTATGGCCT GCTTTACATG TGCAAAGAAG CTAAAGAAAA GGAATAAGCC CTGCCCAGTA   1260
   TGTAGACAAC CAATTCAAAT GATTGTGCTA ACTTATTTCC CC                      1302

### (2) ANGABEN ZU SEQ ID NO: 7:

(i) SEQUENZKENNZEICHEN:
   (A) LÄNGE: 31 Basenpaare
   (B) ART: Nukleinsäure
   (C) STRANGFORM: Einzelstrang
   (D) TOPOLOGIE: linear
(ii) ART DES MOLEKÜLS: DNA-primer
(xi) SEQUENZBESCHREIBUNG: SEQ ID NO: 7:
   CGCGGATCCA TGTGCAATAC CAACATGTCT G 31

### (2) ANGABEN ZU SEQ ID NO: 8:

(i) SEQUENZKENNZEICHEN:
   (A) LÄNGE: 34 Basenpaare
   (B) ART: Nukleinsäure
   (C) STRANGFORM: Einzelstrang
   (D) TOPOLOGIE: linear
(ii) ART DES MOLEKÜLS: DNA-primer
(xi) SEQUENZBESCHREIBUNG: SEQ ID NO: 8:
   CGCGGATCCT CAGGGGAAAT AAGTTAGCAC AATC 34

### (2) ANGABEN ZU SEQ ID NO: 9:

(i) SEQUENZKENNZEICHEN:
   (A) LÄNGE: 33 Basenpaare
   (B) ART: Nukleinsäure
   (C) STRANGFORM: Einzelstrang
   (D) TOPOLOGIE: linear
(ii) ART DES MOLEKÜLS: DNA-primer
(xi) SEQUENZBESCHREIBUNG: SEQ ID NO: 9:
   CGCGGATCCT CACCCTGCCT GATACACAGT AAC 33

### (2) ANGABEN ZU SEQ ID NO: 10:

(i) SEQUENZKENNZEICHEN:
   (A) LÄNGE: 31 Basenpaare
   (B) ART: Nukleinsäure
   (C) STRANGFORM: Einzelstrang
   (D) TOPOLOGIE: linear
(ii) ART DES MOLEKÜLS: DNA-primer
(xi) SEQUENZBESCHREIBUNG: SEQ ID NO: 10:
   CGCGGATCCG GCCAGTATAT TATGACTAAA C                                    31

## Beschreibung

Die Erfindung betrifft ein Verfahren zum Nachweis von hdm-2-spezifischen Antikörpern in Körperflüssigkeiten. Ferner betrifft sie einen hierfür verwendbaren Kit.

Das menschliche Genom umfaßt ein mit hdm-2 (human-double-minute gene 2) bezeichnetes Gen. Dieses Gen weist große Homologien zum entsprechenden mdm-2-Gen (murine-double-minute gene 2) der Maus auf. Das Expressionsprodukt des hdm-2-Gens ist ein Protein, dessen Primärstruktur bekannt ist, und das 491 Aminosäuren aufweist (vgl. Oliner, J.D. et al., Nature 358 (1992), 80-83). Dieses Protein wird nachstehend mit hdm-2 bezeichnet.

Es ist bekannt, daß das hdm-2-Gen bei Sarkomen häufig in amplifizierter Form vorliegt. Es findet sich dann auch eine erhöhte Expression von hdm-2 (Olson et al., Oncogene (1993), 8, S. 2353-2360 ; Olines et al., Nature, Vol. 358, S. 80-83 (1992) ; WO-A-93/20238). Dies kann mit dem Vorliegen spezifischer gegen hdm-2 gerichteter Antikörper einhergehen. Der Nachweis solcher Antikörper könnte daher zur Diagnose spezieller Krebserkrankungen genutzt werden.

Der vorliegenden Erfindung liegt somit die Aufgabe zugrunde, ein Verfahren zum Nachweis von hdm-2-spezifischen Antikörpern bereitzustellen.

Erfindungsgemäß wird dies in einem Verfahren erreicht, bei dem man Trägermaterial-gebundenes hdm-2 und/oder Trägermaterial-gebundene, Bindungsregionen für hdm-2-spezifische Antikörper aufweisende Fragmente davon mit Körperflüssigkeiten inkubiert und die spezifischen an das hdm-2 und/oder die Fragmente gebundenen Antikörper (a)
- mit markierten, gegen die Antikörper (a) gerichteten Antikörpern (b), oder
- mit unmarkierten Antikörpern (b) und letztere mit markierten, gegen die Antikörper (b) gerichteten Antikörpern (c) reagieren läßt.

Der Ausdruck "hdm-2" umfaßt ein hdm-2-Protein mit Wildtyp-Sequenz. Ein solches kann aus Zellen, wie A-204 (humane Rhabdomyosarkom-Zellinie, Tumorbank, Deutsches Krebsforschungszentrum, Heidelberg) isoliert werden. hdm-2 kann auch eine von der Wildtyp-Sequenz abweichende Sequenz haben. Eine solche kann Additionen, Deletionen und/oder Substitutionen von ein oder mehreren Aminosäuren aufweisen. Ferner kann hdm-2 Teil eines Fusionsproteins sein. Ein solches wie auch jedes andere hdm-2 kann aus Zellen isoliert werden, die es nach Genmanipulation exprimieren. Solche Zellen umfassen prokaryotische und eukaryotische Zellen. Beispiele ersterer sind E. Coli-Stämme, wie BL21 (vgl. Studier, F.W. et al., Methods in Enzymology 185 (1990), 60-89), während als letztere insbesondere Säugetier-, Hefe- und Insektenzellen zu nennen sind. Unter Genmanipulationen sind übliche aus dem Stand der Technik bekannte Verfahren zu verstehen, mit denen Nukleinsäuren bestimmter Sequenzen hergestellt, in Zellen eingeführt und exprimiert werden. Der Fachmann kennt hierfür geeignete Materialien, wie Vektoren, und Bedingungen (vgl. z.B. Maniatis, T. et al., Cold Spring Harbor Laboratory, 1982).

Vorliegend wird eine hdm-2-cDNA aus A-204-Zellen (vgl. vorstehend) in üblicher Weise revers transkribiert und in einem gängigen PCR-Verfahren amplifiziert. Die cDNA wird in ihrer Sequenz mit publizierten Daten (z.B. EMBL-Genbank) verglichen und in den bekannten Vektor pet 3d inseriert, wodurch der rekombinante Vektor pet 92/2 erhalten wird. Dieser wird zur Expression von hdm-2 in den Bakterienstamm BL21 (vgl. vorstehend) transformiert. Eine hdm-2-Induktion wird durch Zugabe von IPTG erreicht. Die Bakterien werden sedimentiert und nach Einfrieren und Auftauen einer Lysozym- und DNaseI-Behandlung unterzogen. Die Lysate werden mit Harnstofflösungen verschiedener Konzentrationen inkubiert und nach Zentrifugation wird freigesetztes hdm-2 durch eine Polyacrylamid-Gelelektrophorese und anschließender Elektroelution in reiner Form bereitgestellt. Der Fachmann kennt vorstehende Verfahren und hierzu notwendige Materialien und Bedingungen.

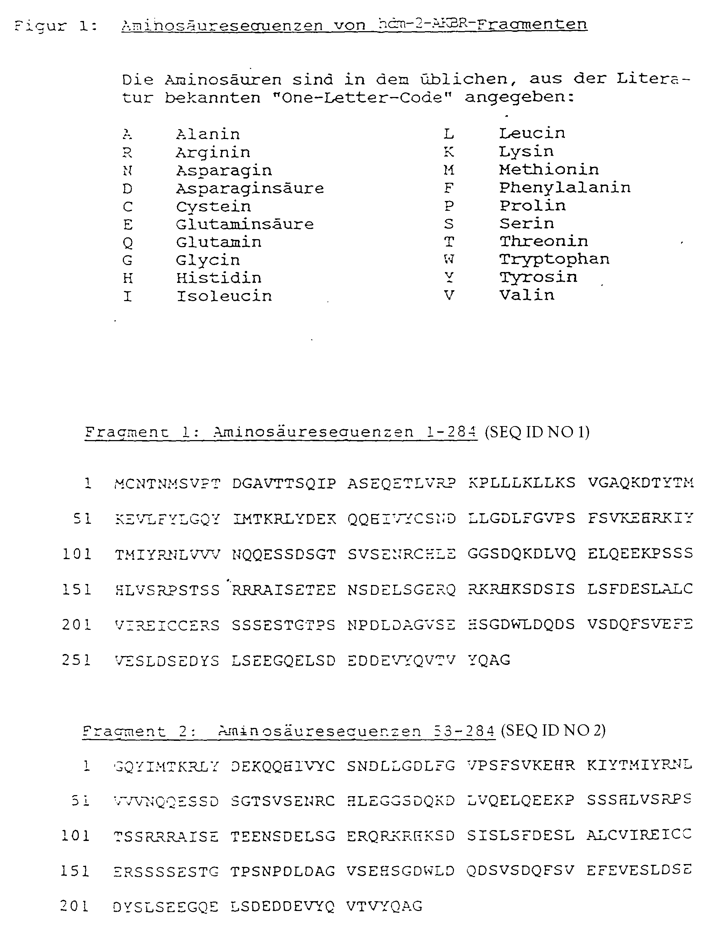

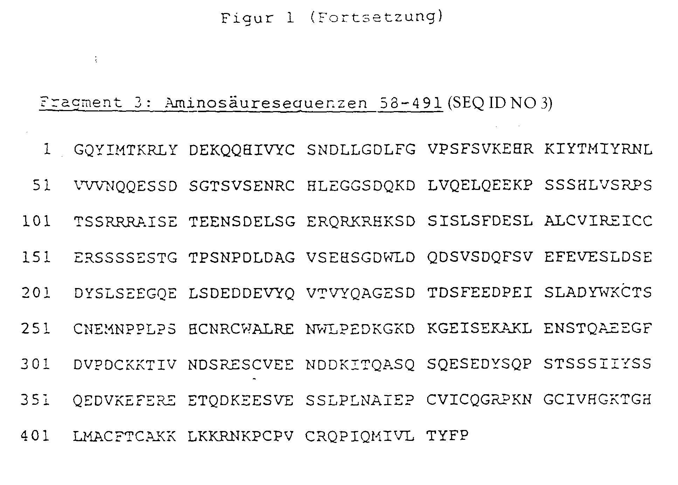

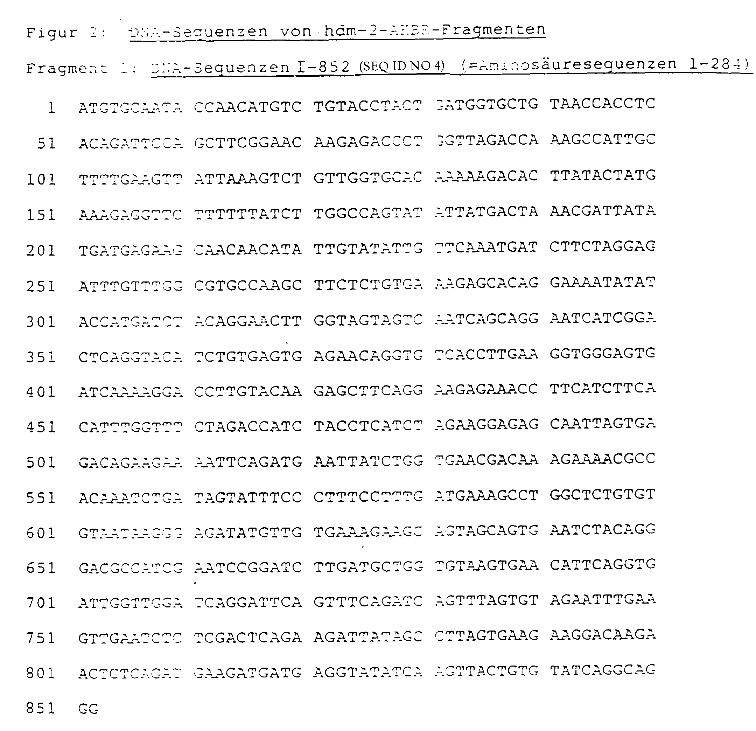

Im Rahmen des erfindungsgemäßen Verfahrens werden hdm-2-Fragmente verwendet, die Bindungsregionen für hdm-2-spezifische Antikörper enthalten. Diese Fragmente werden nachstehend mit hdm-2-AKBR-Fragmente bezeichnet. Vorzugsweise umfassen die hdm-2-AKBR-Fragmente die Aminosäuren 1-284 SEQ ID NO 1, 58-284 SEQ ID NO 2 und 58-491 SEQ ID NO 3 von hdm-2 (vgl. Fig. 1).

Zur Herstellung von hdm-2-AKBR-Fragmenten können mehrere Verfahren verwendet werden. Als günstig hat sich ein Verfahren erwiesen, bei dem man über die Gesamtlänge von hdm-2 verteilt Fragmente konstruiert, wobei jeweils mindestens zwei Fragmente einen überlappenden Bereich aufweisen, die Fragmente mit einem hdm-2-spezifischen Antikörper reagieren läßt und den überlappenden, durch den Antikörper gebundenen Bereich identifiziert und als hdm-2-AKBR-Fragment bereitstellt sowie dieses ggf. als Basis für eine ein- oder mehrfache Wiederholung obigen Zyklus verwendet.

Ausgangspunkt für dieses Verfahren ist eine aus A-204-Zellen erhaltene hdm-2-cDNA (vgl. vorstehend). Von dieser werden über die Gesamtlänge der cDNA verteilt DNA-Fragmente in einem gängigen PCR-Verfahren amplifiziert, wobei jeweils mindestens zwei Fragmente einen überlappenden Bereich aufweisen. Die amplifizierten DNA-Fragmente werden, wie vorstehend beschrieben, in pet 3d inseriert und nach Transformation und IPTG-Induktion im Bakterienstamm BL21 exprimiert. Die erhaltenen hdm-2-Fragmente werden, wie vorstehend für hdm-2 beschrieben, isoliert und einer Polyacrylamid-Gelelektrophorese unterzogen. Dieser folgt eine Westernblot-Analyse, worin markierte, allgemein erhältliche hdm-2-spezifische Antikörper, z.B. JF2, zur Bindung an die hdm-2-Fragmente eingesetzt werden. Durch Bindung eines dieser Antikörper an zwei einen überlappenden Bereich aufweisende hdm-2-Fragmente wird die Bindungsregion des Antikörpers dem überlappenden Bereich zugewiesen. Dieser Bereich wird als mit hdm-2-AKBR bezeichnetes Fragment bereitgestellt. Hierfür wird. z.B. ein gängiges PCR-Verfahren angewandt. Mit dem hdm-2-AKBR-Fragment kann vorstehender Zyklus ein- oder mehrfach wiederholt werden, um die Bindungsregion des hdm-2-spezifischen Antikörpers noch weiter einzugrenzen. Diese Bindungsregion kann bis auf wenige Aminosäuren eingegrenzt werden. Die hierzu u.U. notwendigen kurzen hdm-2-Fragmente können synthetisch hergestellt werden. Dem Fachmann sind die vorstehenden Verfahren und die zu ihrer Durchführung notwendigen Materialien und Bedingungen bekannt.

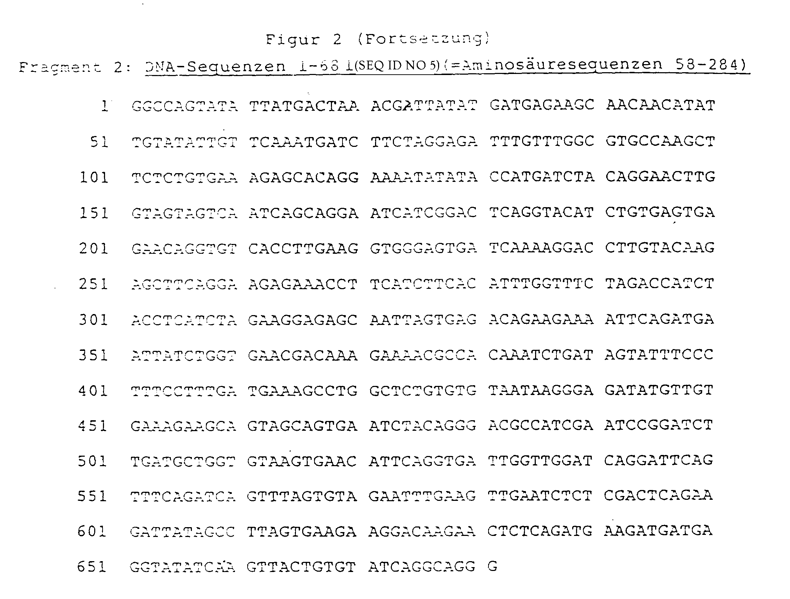

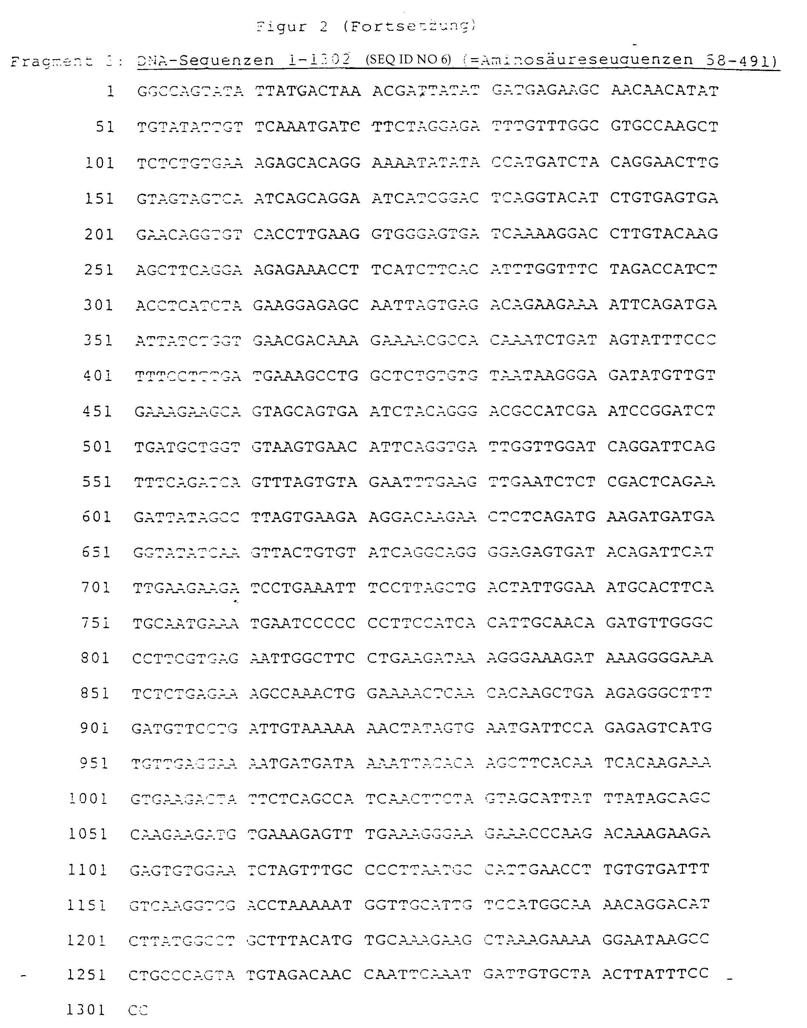

In der vorliegenden Anmeldung werden auch die für hdm-2-AKBR-Fragmente codierenden DNA-Sequenzen gezeigt. Vorzugsweise umfassen diese DNA-Sequenzen die für die Aminosäuren 1-284 SEQ ID NO 4, 58-284 SEQ ID NO 5 und 58-491 SEQ ID NO 6 von hdm-2 codierenden Nukleotide (vgl. Figur 2).

Erfindungsgemäß werden hdm-2 und/oder hdm-2-AKBR-Fragmente an ein Trägermaterial gebunden. Selbstverständlich kann auch ein einzelnes hdm-2-AKBR-Fragment oder dieses zusammen mit hdm-2 gebunden werden. Als Trägermaterial kann jegliches zur Bindung von Proteinen geeignetes Material, insbesondere Mikrotiterplatten, Röhrchen, Mikrokugeln und Objektträger, verwendet werden. Bei sehr kleinen hdm-2-AKBR-Fragmenten, insbesondere Peptiden, ist es ratsam die Bindung an das Trägermaterial über einen üblichen Carrier, z.B. BSA, erfolgen zu lassen. Eine solche Bindung wie auch eine ohne Carrier, kann nach üblichen Verfahren erzielt werden. Vorliegend werden Mikrotiterplatten als Trägermaterial verwendet. Hierzu werden hdm-2 und/oder hdm-2-AKBR-Fragmente in Carbonatpuffer bzw. Harnstoff-Phosphatpuffer aufgenommen, verschieden verdünnt und in die Löcher einer Mikrotiterplatte gegeben. Nach Inkubation über Nacht bei 4°C folgen mehrere Waschschritte in physiologischem Puffer. Die Bindung von hdm-2 und/oder der hdm-2-AKBR-Fragmente ist stabil.

Erfindungsgemäß werden gebundenes hdm-2 und/oder gebundene hdm-2-AKBR-Fragmente mit Körperflüssigkeiten inkubiert. Als solche sind sämtliche Flüssigkeiten gemeint, die aus einem tierischen Körper, insbesondere einem Säugetier und ganz besonders einem Menschen erhalten werden können. Die Flüssigkeiten umfassen vorzugsweise Serum, Lymphe, Speichel und Urin. Ferner gehören zu ihnen auch Flüssigkeiten, die aus festen Geweben, wie Lunge, Gehirn und Knochenmark, sowie aus Tumoren, wie Colorektal-Karzinom und Hepatozell-Karzinom sowie Sarkomen isoliert werden können. Die Inkubation erfolgt nach üblichen Verfahren. Vorliegend werden Seren von Patienten verschieden verdünnt und gebundenem hdm-2 und/oder gebundenen hdm-2-AKBR-Fragmenten in der Mikrotiterplatte zugegeben. Nach 1-stündiger Inkubation bei 37°C folgen mehrere Waschschritte in physiologischem Puffer. Die Bindung von spezifischen Anti-hdm-2-Antikörpern ist stabil.

Erfindungsgemäß werden solche gebundenen Antikörper (nachstehend mit Antikörper (a) bezeichnet)
- mit markierten, gegen die Antikörper (a) gerichteten Antikörpern (b), oder
- mit unmarkierten Antikörpern (b) und letztere mit markierten, gegen die Antikörper (b) gerichteten Antikörpern (c) umgesetzt.

Die Markierung kann radioaktiv oder nicht-radioaktiv sein. Im letzteren Fall werden andere übliche Marker verwendet. Geeignet sind insbesondere Fluoreszenzfarbstoffe, wie z.B. Fluoresceinisothiocyanat, und Enzyme, wie alkalische Phosphatase oder Peroxidase. Als Verstärkersystem kann ein Biotin/Streptavidinkomplex eingesetzt werden. Die Marker sind allgemein erhältlich. Die Konjugierung mit den Antikörpern (b) oder (c) erfolgt nach den Vorschriften des Herstellers. Auch sind bereits markierte Antikörper (b) und (c) allgemein erhältlich.

Die Wahl der geeigneten Antikörper (b), ob markiert oder unmarkiert hängt davon ab, von welchem Tier bzw. welcher Tierart die verwendete Körperflüssigkeit stammt. Handelt es sich beispielsweise um eine Flüssigkeit aus einem Menschen, so werden als Antikörper (b) solche verwendet, die gegen humanes Immunglobulin gerichtet sind. In entsprechender Weise werden, sofern zusätzlich noch Antikörper (c) eingesetzt werden, diese bezüglich des Tiers oder der Tierart ausgewählt, aus der die Antikörper (b) stammen. Die Wahl geeigneter Antikörper ist dem Fachmann bekannt und kann ohne weiteres durchgeführt werden.

Die Umsetzung von gebundenen Antikörpern (a) mit markierten Antikörpern (b) bzw. mit unmarkierten Antikörpern (b) und dann mit markierten Antikörpern (c) kann in üblicher Weise erfolgen. Vorliegend findet die Umsetzung mit den Antikörpern (b) in beiden Alternativen innerhalb von 1 h bei 37°C statt. Nach mehreren Waschvorgängen wird in der ersten Alternative eine dem Marker entsprechende Substratlösung zur Entwicklung der Nachweisreaktion zugegeben. Dies erfolgt gemäß der Anleitung des Herstellers. In der zweiten Alternative werden nach den Waschvorgängen die Antikörper (c) zugegeben. Deren Umsetzung und die Entwicklung der Nachweisreaktion erfolgen in entsprechender Weise.

Das erfindungsgemäße Verfahren weist eine hohe Sensitivität auf. Diese ist besonders hoch, wenn auch die Antikörper (c) eingesetzt werden. Darüberhinaus ist das vorliegende Verfahren rasch in jedem Labor durchführbar. Besondere Sicherheitsvorkehrungen sind hierfür nicht zu treffen. Das erfindungsgemäße Verfahren eignet sich daher besonders zur Durchführung größerer Reihenuntersuchungen.

Erfindungsgemäß wird auch ein Kit bereitgestellt, der zur Durchführung vorstehender Verfahren geeignet ist. Dieser Kit enthält
- Trägermaterial-gebundenes hdm-2 und/oder Trägermaterial-gebundene hdm-2-AKBR-Fragmente und markierte Antikörper (b) sowie übliche Waschpuffer und gegebenenfalls ein der Markierung entsprechendes Substrat oder
- Trägermaterial-gebundenes hdm-2, und/oder Trägermaterial-gebundene hdm-2-AKBR-Fragmente, unmarkierte Antikörper (b) und markierte Antikörper (c) sowie übliche Waschpuffer und gegebenenfalls ein der Markierung entsprechendes Substrat.

Für die Markierung wie auch die anderen Komponenten des Kits gelten die vorstehend für das erfindungsgemäße Verfahren gemachten Ausführungen.

Kurze Erläuterung der Zeichnung:
Figur 1 zeigt die Aminosäuresequenzen von bevorzugten hdm-2-AKBR-Fragmenten (SEQ ID NO 1-3),
Figur 2 zeigt die DNA-Sequenzen der in Figur 1 aufgeführten hdm-2-AKBR-Fragmenten (SEQ ID NO 4-5).

Die vorliegende Erfindung wird durch die Beispiele erläutert.

### Beispiel 1: Expression von hdm-2 und His-hdm-2-Fusionsprotein

### A) Expression von hdm-2

Aus der Zellinie A-204 (vgl. vorstehend) wurde eine hdm-2 cDNA mit einem "hexamer random primer" revers transkribiert und mittels der Oligoprimer "Fhdm-2-B" und "Rhdm-2" in einem gängigen PCR-Verfahren amplifiziert (Sequenz "Fhdm-2-B": CGC GGA TCC ATG TGC AAT ACC AAC ATG TCT G; (SEQ ID NO 7) "Rhdm2": CGC GGA TCC TCA GGG GAA ATA AGT TAG CAC AAT C; (SEQ ID NO 8)) Die amplifizierte Sequenz wurde mit publizierten Daten (EMBL-Genbank) verglichen und über die Restriktionsenzymstelle BamHI in beiden PCR-Primern unter Erhalt des gesamten codierenden Bereichs in dem Vektor pet3d im Bakterienstamm HMS 174 kloniert. Zur Expression von hdm-2 wurde der erhaltene Vektor pet 93/9 in den Bakterienstamm BL21 (vgl. vorstehend) transformiert. Nach Kultivierung der Bakterien bei 37°C bis zu einer optischen Dichte von 0,6 (600 nm) in 100 µg/ml Ampicillin und 30 µg/ml Chloramphenicol enthaltendem LB-Medium wurde eine 3-stündige Induktion von hdm-2 bei 30°C mit 2mM IPTG durchgeführt.

### (B) Expression von His-hdm-2-Fusionsprotein

Aus der Zellinie A-204 (vgl. vorstehend) wurde eine hdm-2 cDNA revers transkribiert und mittels der Oligoprimer "Fhdm-2-B" und "Rhdm-2" (vgl. vorstehend) durch PCR amplifiziert. Die amplifizierte Sequenz wurde über BamHl-Schnittstellen in beiden PCR-Primern in dem bekannten, mit BamHl geöffneten Expressionsvektor pQE-8 kloniert. Die Expression dieses am N-terminalen Ende mit 6 Histidinen verknüpften hdm-2-Proteins erfolgte im Bakterienstamm E.coli SG13009 (vgl. Gottesmann, S. et al., J. Bacteriol. 148, (1981) 265-273). Nach Kultivierung der Bakterien bei 37°C bis zu einer optischen Dichte von 0,6 in LB-Medium mit 100 µg/ml Ampicillin und 25 µg/ml Kanamycin wurde eine 6-stündige Induktion von His-hdm-2-Fusionsprotein bei 37°C mit 1 mM IPTG durchgeführt.

### Beispiel 2: Isolierung und Reinigung von hdm-2 und His-hdm-2-Fusionsprotein

### (A) Isolierung und Reinigung von hdm-2

400 ml Bakterienkultur von Beispiel 1, (A) wurden nach der hdm-2-Induktion durch 10-minütige Zentrifugation bei 500 g sedimentiert, in 50 mM Tris-HCl, pH 8,0, 100 mM NaCl gewaschen und erneut zentrifugiert. Nach Einfrieren und Auftauen wurde das Sediment in 1,6 ml eines Lyse-Puffers resuspendiert (50 mM Tris-HCl, pH 8,0, 10 % Saccharose). Nacheinander wurden hierzu Lysozym (Endkonz. 2mg/ml) und 0,5 M EDTA (Endkonz. 50 mM) zugegeben. Nach 20-minütiger Inkubation auf Eis wurden MgCl₂ (Endkonz. 80 mM) und DNAse I (250 µg) zugegeben, gefolgt von einer 10-minütigen Inkubation bei Raumtemperatur (Endvolumen 6,5 ml). Hierzu wurden 10 µl 0,1M PMSF gegeben und das Gemisch wurde 15 Minuten bei 10000 g, 4°C zentrifugiert. Dieser Zentrifugationsschritt wurde dreimal wiederholt, und zwar jeweils nach Resuspendieren des Sediments und 10-minütiger Inkubation in 1M, 3M bzw. 7M Harnstoff mit PMSF (0,1 M, 10 µl) Der Überstand des letzten Zentrifugationsschritts wurde wie folgt weiter aufgereinigt: Nach Gelelektrophorese in einem 10 % Polyacrylamidgel, Ausschneiden der gewünschten Proteinbande und Elektroelution über Nacht bei 4°C in 25 mM Tris-HCl, 0,2M Glycin, 0,5 % SDS, pH 8,8 in einer allgemein erhältlichen Biotrap-Elutionskammer wurde hdm-2 in reiner Form erhalten.

### (B) Isolierung und Reinigung von His-hdm-2-Fusionsprotein

250 ml Bakterienkultur von Beispiel 1, (B) wurden nach der Induktion des His-hdm-2-Fusionsproteins sedimentiert und einmal in 40ml PBS gewaschen. Je 1g Bakterienpellet wurde in 3 ml Lösung A für 1 min beschallt und anschließend bei RT 8-12h bei mittlerer Geschwindigkeit auf einem Magnetrührer gerührt. Die erhaltene Suspension wurde erneut bei RT für 30 min bei 15000 rpm sedimentiert. 2-6 ml des dabei erhaltenen Überstands wurden auf eine allgemein bekannte Nickel-Chelat-Chromatographiesäule (Ni-NTA-Resin) gegeben. Das Säulenmaterial war zuvor mit 3 Säulenvolumina Lösung A (vgl. nachstehend) gewaschen worden. Die Säule wurde nach Aufladen des Bakterienextrakts nacheinander mit Lösung A bis F gewaschen und zwar mit 2-3 Säulenvolumina der entsprechenden Lösung, solange bis kein Protein mehr eluiert werden konnte. Der Proteingehalt der aufgefangenen Fraktionen wurde durch Extinktionsmessung bei 280 nm photometrisch bestimmt, wobei 1 O.D. ca. 1 mg Protein/ml entsprach. Das His-hdm-2-Fusionsprotein war in den Fraktionen nach Elution mit Lösung D oder E enthalten, wobei in der Regel nur ein sehr geringer Anteil bakterieller Proteine enthalten war. Um diesen Anteil zu entfernen, wurde ggfs. das eluierte Protein nochmals an die Ni-NTA-Resin Säule gebunden, indem der pH-Wert der vereinigten Fraktionen mit der Hauptmenge des zu reinigenden Proteins auf pH 8,0 eingestellt wurde und diese dann nochmals auf die Säule gegeben wurden. Die anschließende Elution erfolgte mit Lösung A bis F, wie vorstehend beschrieben. Die Reinheit und Qualität des eluierten His-hdm-2-Fusionsproteins wurde durch SDS-PAGE geprüft.

Zusammensetzung der verwendeten Lösungen:
- Lösung A:: 6M Guanidinium Hydrochlorid, 0,1M NaH₂PO₄, 10mM β-Mercaptoethanol, 0,01M TrisHCl, pH 8,0
- Lösung B:: 8M Harnstoff, 0,1M NaH₂PO₄, 0,01M TrisHCl, pH 8,0
- Lösung C:: 8M Harnstoff, 0,1M NaH₂PO₄, 0,01M TrisHCl, pH 6,3
- Lösung D:: 8M Harnstoff, 0,1M NaH₂PO₄, 0,01M TrisHCl, pH 5,9
- Lösung E:: 8M Harnstoff, 0,1M NaH₂PO₄, 0,01M TrisHCl, pH 4,5
- Lösung F:: 6M Guanidinium Hydrochlorid, 0,2M Essigsäure

Vorstehendes Verfahren von (B) zeichnet sich durch eine extrem schnelle und effiziente Aufreinigung des exprimierten Proteins aus.

### Beispiel 3: Herstellung von hdm-2-AKBR- und His-hdm-2-AKBR-Fragmenten

### (A) Herstellung eines hdm-2-AKBR-Fragments

Von der aus der Zellinie A-204 (vgl. vorstehend) erhaltenen cDNA wurden zwei sich überlappende DNA-Sequenzen mittels der Oligoprimer-Paare "Fhdm2-B"/"Rhdm2-284" und "Fhdm2-58/Rhdm2-284" in einem gängigen PCR-Verfahren amplifiziert. Die eine DNA-Sequenz codierte für ein hdm-2-Fragment der Aminosäuren 1-284 (SEQ ID NO 4), während die andere für ein hdm-2-Fragment der Aminosäuren 58-284 (SEQ ID NO 5) codierte. Die Sequenzen der verwendeten Oligoprimer waren wie folgt: "Fhdm-2-B" (vgl. vorstehend); "Rhdm-2-284": CGC GGA TCC TCA CCC TGC CTG ATA CAC AGT AAC; (SEQ ID NO 9) "Fhdm-2-58": CGC GGA TCC GGC CAG TAT ATT ATG ACT AAA C (SEQ ID NO 10).

Die amplizierten Sequenzen wurden mit publizierten Daten (EMBL-Genbank) verglichen und über die Restriktionsenzymstelle BamHI in den Vektor pet 3 d (vgl. vorstehend) kloniert. Die Expression der Sequenzen erfolgte nach Transformation und IPTG-Induktion im Bakterienstamm BL21 (vgl. vorstehend). Die exprimierten Sequenzen (hdm-2-Fragmente) wurden, wie in Beispiel 2 (A) für hdm-2 beschrieben, isoliert und einer Polyacrylamid-Gelektrophorese unterzogen. Dieser folgte eine übliche Westernblot-Analyse, worin ein allgemein erhältlicher hdm-2-spezifischer Antikörper zur Bindung an die hdm-2-Fragmente verwendet wurde. Dieser Antikörper zeigte eine Bindung an beide sich überlappende hdm-2-Fragmente. Der überlappende Bereich, d.h. die Aminosäuren 58-284, wurde als Bindungsregion des Antikörpers angesehen.

### (B) Herstellung eines His-hdm-2-AKBR-Fragments

Von der aus der Zellinie A-204 (vgl. vorstehend) erhaltenen cDNA wurden zwei sich überlappende DNA-Sequenzen mittels der Oligoprimer-Paare "Fhdm-2-B"/"Rhdm-2-284" und "Fhdm-2-58"/ "Rhdm-2-284" in einem gängigen PCR-Verfahren amplifiziert.

Die amplizierten Sequenzen wurden mit publizierten Daten (EMBL-Genbank) verglichen und über die Restriktionsenzymstelle BamHI in den Vektor pQE-8 (vgl. vorstehend) kloniert. Die Expression der Sequenzen erfolgte nach Transformation und IPTG-Induktion im Bakterienstamm E.coli SG 13009 (vgl. vorstehend). Die exprimierten Sequenzen (His-hdm-2-Fragmente) wurden, wie in Beispiel 2 (B) für His-hdm-2-Fusionsprotein beschrieben, isoliert und einer Polyacrylamid-Gelektrophorese unterzogen. Dieser folgte eine übliche Westernblot-Analyse, worin ein allgemein erhältlicher hdm-2-spezifischer Antikörper zur Bindung an die His-hdm-2-Fragmente verwendet wurde. Dieser Antikörper zeigte eine Bindung an beide sich überlappende hdm-2-Fragmente. Wie vorstehend in (A) wurde der überlappende Bereich, d.h. die Aminosäuren 58-284, als Bindungsregion des Antikörpers angesehen.

### Beispiel 4: Nachweis von spezifischen hdm-2-Antikörpern im Serum von Patienten durch hdm-2

Zur Durchführung eines ELISA wurde hdm-2 aus Beispiel 2, (B) in Harnstoff-Puffer (3 M Harnstoff, 0.1 M NaH₂PO₄, pH 8.0) aufgenommen. Zur Beschichtung einer 96-Loch-Platte wurden pro Loch je 100 µl mit 20 ng bzw. 8 ng Antigen sowie einmal 1 % BSA als Leerkontrolle einpipettiert. Nach Inkubation über Nacht bei 4°C schlossen sich 3 kurze Waschschritte mit PBS an. Anschließend erfolgte die Blockierung freier Bindungsstellen des polymeren Trägers durch einstündige Inkubation mit 1 % BSA in PBS bei 37°C. Ein zu testendes Serum eines Patienten mit einem kleinzelligen Bronchial-Karzinom wurde in 1:100 Verdünnung (PBS, 1 % BSA, 0,05 % Tween 20) für 1 Stunde bei 37°C auf der Platte inkubiert ("Schachbrett-Titration"). Nach 8 Waschschritten mit PBS (0,05 % Tween 20) wurde ein allgemein erhältlicher Peroxidase-gekoppelter Ziege Anti-Human Antikörper (Verdünnung nach Angabe der Hersteller) zugegeben. Nach dreißigminütiger Inkubation bei 37°C folgten 8 Waschschritte und anschließend die Peroxidase-Nachweisreaktion mit TMB-Entwicklungslösung (50 mM Natriumacetat, 0,4 mM 3,3', 5,5'-Tetramethyl-benzidin-dihydrochlorid, 4,4 mM H₂O₂) innerhalb 30 Minuten bei Raumtemperatur. Nach dem Abstoppen der Reaktion mit 2 M HCl wurde die Farbintensität photometrisch bei 450 nm bestimmt. Absorbtionswerte des mehr als doppelten der BSA-Kontrolle wurden als positive Reaktion gewertet.

Die Tabelle zeigt die Daten eines entsprechend durchgeführten ELISA im Vergleich zur Bestimmung spezifischer Antikörper mittels Immunoblot. Die nahezu vollständige Übereinstimmung beider Verfahren weist den Anti-hdm-2 ELISA als geeignetes Verfahren zur Reihentestung klinischen Materials aus. Ferner zeigt der ELISA eine größere Sensitivität als der Immunoblot, wodurch die Eignung des ELISA als Erstuntersuchungsverfahren unterstrichen wird.

**Tabelle:**

| Vergleich des Nachweises von hdm-2-Antikörpern in Seren von Patienten mit Bronchialkarzinom durch Immunblot und ELISA mit rekombinatem hdm-2 | | | | | | |
|---|---|---|---|---|---|---|
| Serum-Code | 1000 | 1001 | 1100 | 1502 | 1503 | 2102 |
| Immunblot | - | - | - | + | + | + |
| ELISA | - | - | - | + | + | + |
| Legende: Immunblot: | | | | | | |
| +: sichtbare Bande auf der Höhe von 90 kD Molekulargewicht bei einer Serumverdünnung von 1:100, Inkubation mit Peroxidase-gekoppeltem, human-spezifischen Ziegen-Antikörper (Dianova, 1:20000) und Peroxidase-Nachweisreaktion. | | | | | | |
| -: kein sichtbares Signal | | | | | | |
| ELISA: | | | | | | |
| + : Absorptionswert des wenigstens 2-fachen des BSA-Kontrollwerts | | | | | | |
| - : Absorptionswert ≤ des BSA-Kontrollwerts | | | | | | |

Die Tabelle zeigt, daß die Ergebnisse von Immunblot und ELISA übereinstimmen, wobei die größere Sensitivität des ELISA auf eine Eignung als Erstuntersuchungmethode hindeutet.

## Patentansprüche

1. Verfahren zum Nachweis von "human-double-minute gene 2" (hdm-2)-spezifischen Antikörpern in Körperflüssigkeiten, dadurch gekennzeichnet, daß man Trägermaterial-gebundenes hdm-2 und/oder Trägermaterial-gebundene, Bindungsregionen für hdm-2-spezifische Antikörper aufweisende Fragmente davon mit Körperflüssigkeiten inkubiert und die spezifischen, an das hdm-2 und/oder die Fragmente gebundenen Antikörper (a)
- mit markierten, gegen die Antikörper (a) gerichteten Antikörpern (b), oder
- mit unmarkierten Antikörpern (b) und letztere mit markierten, gegen die Antikörper (b) gerichteten Antikörpern (c) reagieren läßt.

2. Verfahren nach Anspruch 1, dadurch gekennzeichnet, daß die Körperflüssigkeiten Serum, Lymphe, Speichel und Urin sowie aus festen Geweben und Tumoren erhaltene Flüssigkeiten umfassen.

3. Verfahren nach Anspruch 1 oder 2, dadurch gekennzeichnet, daß das hdm-2 durch Expression einer cDNA-Sequenz erhalten ist.

4. Verfahren nach Anspruch 1 oder 2, dadurch gekennzeichnet, daß die hdm-2-Fragmente die Aminosäuren 1-284 (SEQ ID NO 1), 58-284 (SEQ ID NO 2) und 58-491 (SEQ ID NO 3) von hdm-2 umfassen.

5. Verfahren nach einem der Ansprüche 1 - 4, dadurch gekennzeichnet, daß das Trägermaterial Mikrotiterplatten, Röhrchen, Mikrokugeln und Objektträger umfaßt.

6. Verfahren nach einem der Ansprüche 1 - 5, dadurch gekennzeichnet, daß die Antikörper (b) in der ersten Alternative und die Antikörper (c) in der zweiten Alternative mit einem Enzym markiert sind.

7. Verfahren nach einem der Ansprüche 1 - 5, dadurch gekennzeichnet, daß die Antikörper (b) in der ersten Alternative und die Antikörper (c) in der zweiten Alternative mit einem Fluoreszenzfarbstoff markiert sind.

8. Verfahren nach einem der Ansprüche 1 - 5, dadurch gekennzeichnet, daß die Antikörper (b) in der ersten Alternative und die Antikörper (c) in der zweiten Alternative radioaktiv markiert sind.

9. Kit, enthaltend
- Trägermaterial-gebundenes hdm-2 und/oder Trägermaterial-gebundene, Bindungsregionen für hdm-2-spezifische Antikörper aufweisende Fragmente davon und markierte Antikörper (b) nach Anspruch 1 sowie übliche Waschpuffer und ggfs. ein der Markierung entsprechendes Substrat oder
- Trägermaterial-gebundene hdm-2 und/oder Trägermaterial-gebundene, Bindungsregionen für hdm-2-spezifische Antikörper aufweisende Fragmente davon und unmarkierte Antikörper (b) und markierte Antikörper (c) nach Anspruch 1 sowie übliche Waschpuffer und ggf. ein der Markierung entsprechendes Substrat.

## Claims

1. A method of identifying "human-double-minute gene 2" (hdm-2)-specific antibodies in body fluids, characterized in that carrier material-bound hdm-2 and/or carrier material bound fragments thereof including binding sites for hdm-2-specific antibodies are incubated with body fluids and the specific antibodies (a) bound to hdm-2 and/or the fragments are allowed to react
- with labeled antibodies (b) directed against antibodies (a) or
- with unlabeled antibodies (b) and the latter with labeled antibodies (c) directed against antibodies (b).

2. The method according to claim 1, characterized in that the body fluids comprise serum, lymph, saliva and urine as well as fluids obtained from solid tissues and tumors.

3. The method according to claim 1 or 2, characterized in that the hdm-2 is obtained by expression of a cDNA sequence.

4. The method according to claim 1 or 2, characterized in that the hdm-2 fragments comprise the amino acids 1-284 (SEQ ID No. 1), 58-284 (SEQ ID No. 2), and 58-491 (SEQ ID No. 3) of hdm-2.

5. The method according to any one of claims 1 to 4, characterized in that the carrier material comprises microtiter plates, small tubes or pipes, microspheres and slides.

6. The method according to any one of claims 1 to 5, characterized in that antibodies (b) in the first alternative and antibodies (c) in the second alternative are labeled with an enzyme.

7. The method according to any one of claims 1 to 5, characterized in that antibodies (b) in the first alternative and antibodies (c) in the second alternative are labeled with a fluorescent dye.

8. The method according to any one of claims 1 to 5, characterized in that antibodies (b) in the first alternative and antibodies (c) in the second alternative are labeled radioactively.

9. Kit containing
- carrier material-bound hdm-2 and/or carrier material-bound fragments thereof including binding sites for hdm-2-specific antibodies and labeled antibodies (b) according to claim 1 as well as conventional wash buffers and optionally a substrate corresponding to the labeling or
- carrier material-bound hdm-2 and/or carrier material-bound fragments thereof including binding sites for hdm-2-specific antibodies and unlabeled antibodies (b) and labeled antibodies (c) according to claim 1 as well as conventional wash buffers and optionally a substrate corresponding to the labeling.

## Revendications

1. Méthode de détection d'anticorps spécifiques du gène "human-double-minute 2" (hdm-2) dans des liquides corporels, caractérisée en ce qu'on fait incuber avec des liquides corporels le gène hdm-2 lié à un support et/ou des fragments de ce gêne liés à un support, présentant des sites de liaison pour des anticorps spécifiques du gène hdm et on fait réagir les anticorps spécifiques (a) liés au gène hdm-2 et/ou aux fragments
- avec des anticorps marqués (b) dirigés contre les anticorps (a) ou bien
- avec des anticorps (b) non marqués et on fait réagir ces derniers avec des anticorps marqués (c) dirigés contre les anticorps (b).

2. Méthode suivant la revendication 1, caractérisée en ce que les liquides corporels comprennent le sérum, la lymphe, la salive et l'urine ainsi que des liquides obtenus à partir de tissus solides et de tumeurs.

3. Méthode suivant la revendication 1 ou 2, caractérisée en ce que le gène hdm-2 est obtenu par expression d'une séquence d'ADNc.

4. Méthode suivant la revendication 1 ou 2, caractérisée en ce que les fragments de hdm-2 comprennent les aminoacides 1-284 (SEQ ID N° 1), 58-284 (SEQ ID N° 2) et 58-491 (SEQ ID N° 3) de hdm-2.

5. Méthode suivant l'une des revendications 1 à 4, caractérisée en ce que le support comprend des plaques de microtitrage, des petits tubes, des microsphères et des porte-objets.

6. Méthode suivant l'une des revendications 1 à 5, caractérisée en ce que les anticorps (b) dans la première variante et les anticorps (c) dans la seconde variante sont marqués avec un enzyme.

7. Méthode suivant l'une des revendications 1 à 5, caractérisée en ce que les anticorps (b) dans la première variante et les anticorps (c) dans la seconde variante sont marqués avec un colorant fluorescent.

8. Méthode suivant l'une des revendications 1 à 5, caractérisée en ce que les anticorps (b) dans la première variante et les anticorps (c) dans la seconde variante sont marqués par la radioactivité.

9. Kit contenant
- le gène hdm-2 lié à un support et/ou des fragments de ce gène liés à un support, présentant des sites de liaison pour des anticorps spécifiques de hdm-2, et des anticorps (b) marqués suivant la revendication 1, ainsi qu'un tampon de lavage classique et le cas échéant un substrat correspondant au marquage ou bien
- le gène hdm-2 lié à un support et/ou des fragments de ce gène liés à un support, présentant des sites de liaison pour des anticorps spécifiques du gène hdm-2, et des anticorps (b) non marqués et des anticorps (c) marqués suivant la revendication 1 ainsi que les tampons de lavage classiques et le cas échéant un substrat correspondant au marquage.
